# EUROPÄISCHE PATENTSCHRIFT

(11) **EP 3 374 353 B1**
(45) Veröffentlichungstag und Bekanntmachung des Hinweises auf die Patenterteilung: **13.02.2019**
(21) Anmeldenummer: 17735072.5
(22) Anmeldetag: 28.06.2017
(51) Int. Cl.: C07D 301/12, C07D 225/02, C07C 45/58, C07C 249/08, B01J 38/64, B01J 38/66, B01J 23/30

(54) **VERFAHREN ZUR REAKTIVIERUNG EINES HOMOGENEN OXIDATIONSKATALYSATORS**
METHOD FOR REACTIVATION OF A HOMOGENEOUS OXIDATION CATALYST
PROCÉDÉ POUR LA RÉACTIVATION D'UN CATALYSEUR D'OXYDATION HOMOGÈNE

(30) Priorität: 30.06.2016 EP 16177260
(43) Veröffentlichungstag der Anmeldung: 19.09.2018
(73) Patentinhaber: Evonik Degussa GmbH, 45128 Essen (DE)
(72) Erfinder: MEIER, Ralf, 44265 Dortmund (DE); BAJUS, Stephanie, 63450 Hanau (DE); DÖRING, Jens, 44141 Dortmund (DE)
(86) Internationale Anmeldenummer: PCT/EP2017/065957
(87) Internationale Veröffentlichungsnummer: WO 2018/002114

(56) Entgegenhaltungen:
- EP-A1- 1 411 051
- EP-A1- 2 980 069
- EP-A2- 2 946 831
- GB-A- 894 592
- US-A- 3 231 329
- US-A- 4 197 161
- CHOWDHURY, S.R. ET AL.: "Recovery of Homogeneous Polyoxometallate Catalysts from Aqueous and Organic Media by a Mesoporous Ceramic Membrane without Loss of Catalytic Activity", CHEMISTRY - A EUROPEAN JOURNAL., Bd. 12, Nr. 11, 2006, Seiten 3061-3066, XP055200347, ISSN: 0947-6539, DOI: 10.1002/chem.200501021
- KAMATA, K. ET AL.: "Epoxidation of Alkenes with Hydrogen Peroxide Catalyzed by Selenium-Containing Dinuclear Peroxotungstate and Kinetic, Spectroscopic, and Theoretical Investigation of the Mechanism", INORGANIC CHEMISTRY, Bd. 49, Nr. 5, 2010, Seiten 2471-2478, XP055330858, ISSN: 0020-1669, DOI: 10.1021/ic902381b
- MIZUNO, N. ET AL.: "Molecular design of selective oxidation catalyst with polyoxometalate", CATALYSIS TODAY, Bd. 117, Nr. 1-3, 2006, Seiten 32-36, XP027976161, ISSN: 0920-5861 [gefunden am 2006-09-30]
- HAIMOV, A. ET AL.: "Alkylated Polyethyleneimine/Polyoxometalate Synzymes as Catalysts for the Oxidation of Hydrophobic Substrates in Water with Hydrogen Peroxide", JOURNAL OF THE AMERICAN CHEMICAL SOCIETY, Bd. 126, Nr. 38, 2004, Seiten 11762-11763, XP055330819, ISSN: 0002-7863, DOI: 10.1021/ja046349u

## Beschreibung

Die vorliegende Erfindung betrifft ein Verfahren zur Reaktivierung eines homogenen Oxidationskatalysators. Des Weiteren betrifft die Erfindung ein Verfahren zur Oxidation organischer Verbindungen. Darüber hinaus wird ein Verfahren zur Synthese von Laurinlactam sowie Polyamid 12 beschrieben.

Cyclododecanon (CDON) wird für die Synthese von Laurinlactam eingesetzt. Das Lactam wiederum eignet sich für die Herstellung von Polyamid 12. Die Herstellung von CDON kann ausgehend von Cyclododecatrien (CDT) erfolgen. Zunächst kann eine Selektivhydrierung von Cyclododecatrien (CDT) zu Cyclododecen (CDEN) vorgenommen werden. Anschließend folgen eine Epoxidierung von CDEN zu Mono-Epoxycyclododecan (CDANepoxid) und die Umlagerung von CDANepoxid zu Cyclododecanon (CDON).

Die Epoxidierung des CDEN kann beispielsweise in einem Zweiphasensystem in Gegenwart eines homogenen Katalysatorsystems erfolgen. Gemäß EP-A-2946831 (US 2015/0328619) wird das epoxidierte Produkt vom verbleibenden Reaktionsgemisch an einer Membran abgetrennt. Das Katalysatorsystem verbleibt im Retentant und kann dem Reaktionsgemisch im Kreislauf als kontinuierliches Verfahren wieder zugeführt werden.

Hierbei ist allerdings beobachtet worden, dass der Umsatz bei konstanter Katalysatorkonzentration im Laufe der Zeit abnimmt. Ein abnehmender Umsatz lässt sich durch die Zufuhr frischen Katalysators kompensieren. Folglich ist beobachtet worden, dass sich der Katalysator insbesondere bei kontinuierlichen Verfahren immer weiter aufkonzentriert, um einen konstanten Umsatz zu halten. Als Folge wandelt sich der Katalysator in weniger reaktive Spezies um. Weiterhin fällt der Katalysator an vielen Stellen der Apparatur oder Anlage aus. Der Feststoff stört die Reaktionsführung und verstopft eingesetzte Membranfilter.

Das Hinzuführen neuen Katalysators hat allerdings den Nachteil, dass der Verbrauch an Katalysator ansteigt. Neben steigenden Kosten führt dies vor allem zu einer erhöhten Belastung der Umwelt.

Es bestand nunmehr die Aufgabe, ein verbessertes Verfahren zur Oxidation organischer Verbindungen zur Verfügung zu stellen. Hierbei sollte der Umsatz der eingesetzten organischen Verbindung möglichst konstant gehalten werden. Weiterhin sollte insbesondere die Menge an neuem Katalysator verringert werden, um die Umweltbelastung nachhaltig zu vermeiden. Darüber hinaus sollte die Bildung von ausgefallenem Katalysator vermieden werden.

Demgemäß wurde ein neues Verfahren gemäß Anspruch 1 gefunden, das eine Reaktivierung des eingesetzten Katalysators erlaubt. Hierdurch kann die Menge an frischem Katalysator verringert werden oder vollständig auf frischen Katalysator verzichtet werden. In dem Verfahren wird eine ungesättigte organische Verbindung in einem Reaktionsgemisch unter Erhalt eines oxidierten Produkts oxidiert. Das Reaktionsgemisch umfasst ein homogenes Katalysatorsystem, welches die Oxidation der organischen Verbindung katalysiert, sowie mindestens ein Peroxid. Das Katalysatorsystem umfasst mindestens ein Derivat eines Metalls in seiner höchsten Oxidationsstufe, wobei das Derivat ausgewählt ist aus H₂WO₄ und H₂MoO₄ bzw. deren Alkali- oder Erdalkalisalze. Zudem ist mindestens ein Phosphorderivat, nämlich Phosphorsäure oder deren Salz, umfasst.

Die Reaktivierung des Katalysatorsystems erfolgt durch Zugabe mindestens einer wässrigen Base auf einen pH-Wert ≥ 4, vorzugsweise ≥ 4,5, bevorzugt ≥ 7und besonders bevorzugt ≥ 11.

Durch das erfindungsgemäße Verfahren ist es möglich, das Ausfallen des Katalysatorsystems zu vermeiden. Das Aufkonzentrieren des Katalysatorsystems kann unterbunden werden, wobei im Vergleich zum Stand der Technik ein gleichhoher Umsatz realisiert werden kann.

Weitere Ausgestaltungen der Erfindung finden sich in den weiteren Ansprüchen. Oxidationen im Sinne der Erfindung sind Epoxidierungen.

Unter dem Begriff organische Verbindungen werden im Rahmen dieser Erfindung Verbindungen verstanden, die mindestens Kohlenstoffatome enthalten, linear oder cyclisch sind und eine oder mehrere Doppelbindungen enthalten können. Die organischen Verbindungen sind ungesättigt. Vorzugsweise enthalten die ungesättigten organische Verbindungen C=C-Doppelbindungen, wobei bevorzugt keine Dreifachbindungen enthalten sind. Bevorzugt sind ungesättigte Verbindungen mit sechs bis zwölf Kohlenstoffatomen, wobei diese besonders bevorzugt cyclisch sind, jeweils insbesondere umfassend C=C-Doppelbindungen. Ganz besonders bevorzugt sind cyclische, ungesättigte C12-Verbindungen, weiter bevorzugt sind cyclische C12-Verbindungen mit C=C-Doppelbindungen, insbesondere CDEN. Aus CDEN entsteht durch die Reaktion CDANepoxid.

Geeignete Peroxide sind dem Fachmann bekannt. Hierzu zählen 3-Chlorperoxybenzoesäure, Peroxybenzoesäure, Peroxyessigsäure, Peroxybenzimidsäure, tert-Butylhydroperoxid, Dimethyldioxiran, Kaliumhydrogenperoxomonosulfat und Wasserstoffperoxid, wobei Wasserstoffperoxid bevorzugt ist.

Geeignete Katalysatorsysteme enthalten Wolfram oder Molybdän, wie sie beispielsweise in WO 00/44704 A1 (AU 2299400 A) oder DE 3027349 A1 (GB 2055821 B) beschrieben sind. Das Katalysatorsystem kann die katalytisch wirkende Substanz selbst sein oder eine Mischung mit Wasser oder organischen Lösemitteln. Die organische Verbindung kann als organisches Lösemittel fungieren.

Das Katalysatorsystem umfasst ein Derivat eines Metalls, das ausgewählt ist aus Wolfram und Molybdän. Das Derivat sind die Sauerstoffsäuren H₂WO₄ und H₂MoO₄ bzw. deren Alkali- oder Erdalkalisalze. Beispielsweise umfassen bekannte Epoxidierungskatalysatoren Wolframat oder Molybdat, insbesondere Natriumwolframat, Na₂WO₄, oder Natriummolybdat, Na₂MoO₄.

Diese Verbindungen werden meist in situ in die katalytisch aktive Verbindung umgewandelt. Dies erfolgt durch Umsetzung mit einem Derivat von Phosphor, sowie zusätzlich Silicium und/oder Arsen. Besonders eignet sich hierfür ein Oxid, eine Sauerstoffsäure, ein Salz einer Sauerstoffsäure, ein Sulfid, ein Chlorid, ein Oxychlorid oder ein Fluorid von Silicium und/oder Arsen.

Bevorzugt umfasst das Katalysatorsystem deshalb eine katalytisch aktive Verbindung, die durch Umsetzung eines Derivats von Wolfram oder Molybdän mit einem Derivat von Phosphor sowie ggf. Silicium oder Arsen erhalten wird. In einer ganz besonders bevorzugten Ausführungsform wird die katalytisch aktive Übergangsmetallverbindung in situ durch Umsetzung von Natriumwolframat mit Phosphorsäure gebildet.

In einer besonders bevorzugten Ausführungsform der vorliegenden Erfindung umfasst das Katalysatorsystem demnach Phosphorsäure und ein Derivat eines Metalls, das ausgewählt ist aus Wolfram und Molybdän, welches in Verbindung mit einem Phasentransferreagenz eingesetzt wird (das Phasentransferreagenz bildet somit nicht einen Bestandteil des Katalysatorsystems).

Das Reaktionsgemisch umfasst vorzugsweise zwei nicht oder schwer mischbare flüssige Phasen. Eine der Phasen enthält im Wesentlichen Wasser (wässrige Phase). Daneben kann diese Phase das Peroxid, das Katalysatorsystem, Phasentransferreagenz und Spuren der organischen Verbindung sowie ihrer Folgeprodukte enthalten. Die andere Phase (organische Phase) enthält typischerweise im Wesentlichen das Reaktionsprodukt der Oxidation. Daneben kann diese Phase die organische Verbindung sowie das Katalysatorsystem und das Phasentransferreagenz enthalten. Insofern ist das Reaktionsgemisch vorzugsweise ein Mehrphasensystem umfassend eine wässrige und eine organische Phase, wobei weiterhin ein Phasentransferreagenz enthalten ist. Die beschriebenen Katalysatoren werden häufig in Verbindung mit einem Phasentransferreagenz eingesetzt, mit dessen Hilfe der an sich wasserlösliche Katalysator in die organische Phase überführt werden kann.

Als Phasentransferreagenz eignen sich hierbei insbesondere ternäre und quartäre Ammoniumverbindungen sowie Esterquats, wie sie beispielsweise in EP-A-2946831 beschrieben sind. Bevorzugt sind Amine oder Ammoniumsalze, wobei quartäre Ammoniumsalze besonders bevorzugt sind. Ein Beispiel für ein geeignetes Phasentransferreagenz ist das unter dem Namen Alamin erhältliche Trioctylamin. Die Octylreste können zumindest teilweise durch Decylreste ersetzt worden sein. Beispiele für quartäre Ammoniumverbindungen sind Trialkylammoniummethyl-salze, wobei die Alkylketten aus jeweils sechs bis zwölf C-Atomen bestehen. Das Trialkylammoniummethyl-sulfat ist ein ganz besonders bevorzugtes Phasentransferreagenz dieser Erfindung.

Die Reaktionsgeschwindigkeit der Oxidation ist, soweit eine wässrige Phase umfasst ist, abhängig vom pH-Wert. Insofern ist es bevorzugt, die Oxidation bei einem pH ≤ 4, vorzugsweise in einem Bereich von 1,5 bis 4, stattfinden zu lassen. Die Einstellung des pH-Werts erfolgt vorzugsweise mit mindestens einer anorganischen Säure, die bei 25 °C einen pKₛ-Wert (bzw. einen pKₛ₁-Wert) von 2,5 oder weniger aufweist. Geeignete Säuren werden ausgewählt aus Phosphorsäure, Salpetersäure, Schwefelsäure, Salzsäure, Perchlorsäure und ihren Mischungen, wobei bevorzugt Phosphorsäure, Schwefelsäure oder ihre Mischungen und besonders bevorzugt Schwefelsäure eingesetzt wird.

Die Oxidation kann in einem organischen Lösemittel durchgeführt werden. Alternativ kann die Oxidation ohne Lösemittel erfolgen, da die organische Verbindung selbst als Lösemittel fungieren kann.

Die Reaktivierung des Katalysatorsystems erfolgt mittels wässriger Base. Bevorzugte Basen sind aus Ammoniak, Alkalihydroxiden oder Mischungen daraus ausgewählt. Natrium und Kalium sind bevorzugte Alkalimetalle, wobei Natrium besonders bevorzugt ist. Geeignete Basen sind Natronlauge oder Kalilauge. Die Lösung der Base wird derart zugegeben, dass der gewünschte pH-Wert eingestellt ist. Durch eine chemische Reaktion wird der Katalysator reaktiviert.

Bevor die Reaktivierung vorgenommen wird, wird die oxidierte organische Verbindung (Produkt) und gegebenenfalls das Lösemittel vom Katalysatorsystem abgetrennt. Dadurch wird das Katalysatorsystem aufkonzentriert. Dies kann beispielsweise mittels Destillation, Extraktion, Kristallisation oder Membranfiltration erfolgen, wobei die Membranfiltration bevorzugt ist. Der Fachmann ist mit derartigen Abtrennungsmethoden vertraut. Hinsichtlich der Membranfiltration wird insbesondere auf EP-A-2946831 verwiesen. Die Membranfiltration weist den Vorteil auf, dass die Reaktion und Reaktivierung im Kreislauf als kontinuierliches Verfahren durchgeführt werden kann. Das aufkonzentrierte Katalysatorsystem (Retentat) wird im Anschluss mit der wässrigen Base vermischt.

Nach der Reaktivierung wird der Katalysator dem Reaktionsgemisch wieder zugeführt (kontinuierliche Verfahrensweise). Alternativ kann der Katalysator zwischengelagert und für spätere Reaktionen eingesetzt werden (Batch-Verfahrensweise), wobei eine kontinuierliche Verfahrensweise bevorzugt ist. Die Zuführung kann erfolgen, indem diejenige Phase zurückgeführt wird, in der sich der Katalysator befindet. Die Verteilung des metallischen Katalystors in den jeweiligen Phasen kann durch den pH-Wert gesteuert werden.

Nach der Reaktivierung und vor der Zuführung zu einem Reaktionsgemisch erfolgt vorzugsweise die Einstellung des pH-Werts in der sich ergebenden wässrigen Phase. Diese Maßnahme stellt sicher, dass keine Zersetzung des Peroxids, insbesondere des Wasserstoffperoxids, im Reaktionsgemisch erfolgt. Der pH-Wert sollte vorteilhafterweise in einem Bereich von < 7, vorzugsweise 1,5 - 4 eingestellt werden. Die Einstellung des pH-Werts wird vorzugsweise mit einer anorganischen Säure vorgenommen, die einen pKₛ-Wert (bzw. einen pKₛ₁-Wert) bei 25 °C von 2,5 oder weniger aufweist. Geeignete Säuren werden ausgewählt aus Phosphorsäure, Salpetersäure, Schwefelsäure, Salzsäure und Perchlorsäure, wobei Phosphorsäure, Schwefelsäure und ihre Mischungen bevorzugt sind und Schwefelsäure besonders bevorzugt ist. Ist der pH-Wert eingestellt, kann das Katalysatorsystem dem Reaktionsgemisch wieder zugeführt werden. Hierbei ist es vorteilhaft, eine ggf. vorhandene organische Phase abzutrennen.

Bevor das Reaktionsgemisch im Falle der Membranfiltration der Membran zugeführt wird, erfolgt, sofern zwei flüssige Phasen vorhanden sind, vorzugsweise ihre Trennung.

Diese kann beispielsweise mittels Phasentrennbehälter durchgeführt werden. Insofern wird das Reaktionsgemisch nicht als zweiphasiges, sondern als einphasiges Gemisch auf die Membran gegeben. Vorzugsweise wird die nicht-wässrige (organische) Phase per Membran getrennt. Der Katalysator kann auch aus der wässrige Phase mittels Membrantechnik abgetrennt werden. Mithin werden wässrige und organische Phase getrennt voneinander mit einer jeweiligen Membran von Katalysator bereinigt. Für die wässrige Phase kann eine andere Membran verwendet werden als für die organische Phase. Das Retentat enthaltend die katalysatorhaltige organische Phase wird im Anschluss der Reaktivierung zugeführt.

Für die wässrige Phase wird vorzugsweise eines der folgenden Membranmaterialen verwendet: Polyamide, aromatische Polyamide, Polysulfone, Polyethersulfone, hydrophobierte Polyethersulfone, sulfonierte Polyethersulfone, Celluloseactat, Polypiperazin und Polyvinylidenfluorid. Für die Auftrennung der organischen Phase sollte hingegen vorzugsweise eine Membran auf Basis von Siliconacrylat und/oder Polydimethylsiloxan (PDMS) und/oder Polyimid verwendet werden. Das aus beiden Phasen aufkonzentrierte Katalysatorsystem kann vereinigt werden.

Sofern keine Phasentrennung des Reaktionsgemischs eintreten sollte, sind dem Fachmann Maßnahmen zur Phasentrennung wie Erhöhung der Polarität der wässrigen Phase oder Änderung der Dichte einer Phase bekannt.

Mit dem Membranverfahren wird das Katalysatorsystem im Retentat angereichert. Unter "Retentat" versteht der Membrantechniker den Abfluss von der Membran, der vor der Membran abgezogen wird. Das Material, welches die Membran überwindet, wird "Permeat" genannt und hinter der Membran abgezogen. Die Menge an zurückgehaltenem Katalysator kann dabei anhand der Menge an zurückgehaltenem Übergangsmetall nachgewiesen werden. Das Verfahren erlaubt es insbesondere, mehr als 50 % des Übergangsmetalls, bezogen auf die Gesamtmenge an Übergangsmetall im Reaktionsgemisch vor der Filtration, im Retentat zurückzuhalten. Bevorzugt werden mehr als 70 % des Übergangsmetalls zurückgehalten, besonders bevorzugt mehr als 90 %. Falls ein Phasentransferreagenz eingesetzt wird, wird dieses in der Regel einen anderen Rückhalt erzielen als das Übergangsmetall. Dennoch wird es im Retentat angereichert.

Deswegen wird das gesamte Katalysatorsystem zumindest teilweise im Retentat angereichert. Das Übergangsmetall kann durch ICP-MS (Massenspektroskopie mit induktiv gekoppeltem Plasma) oder RFA (Röntgenfluoreszenzanalyse) nachgewiesen werden.

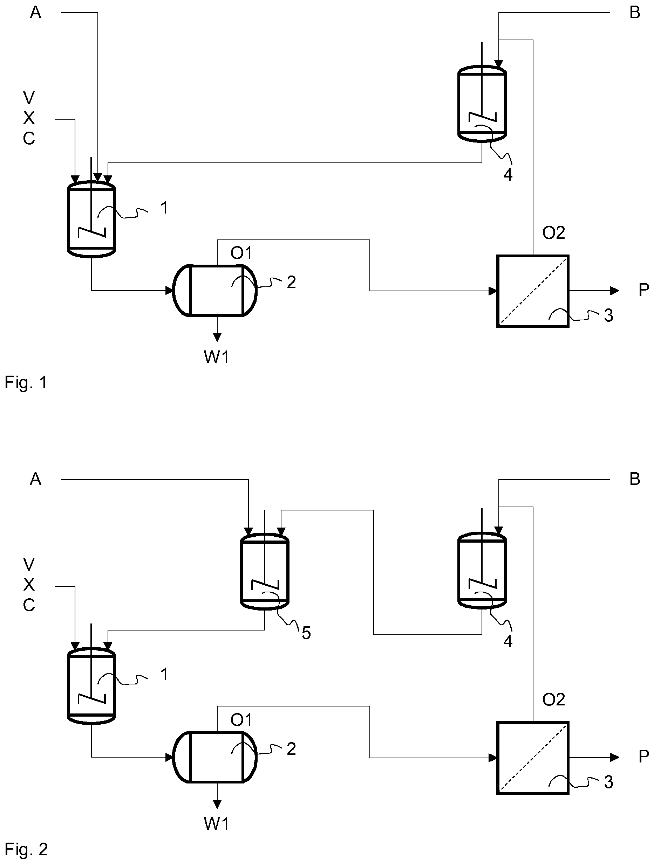

Die Figuren 1 bis 4 veranschaulichen den Prozessverlauf einer kontinuierlichen Verfahrensweise mittels Membranfiltration. Diese Verfahrensführungen stellen besonders bevorzugte Ausführungsformen der Erfindung dar. Die organische Verbindung V, vorzugsweise eine ungesättigte cyclische C6- bis C12-Verbindung, insbesondere CDEN, das Peroxid X und das Katalysatorsystem C in Form einer wässrigen Lösung werden zusammen mit einem Phasentransferreagenz in einen Rührkessel (1) gegeben. Der pH-Wert wird mittels Säure - soweit erforderlich - auf einen bevorzugten Wert ≤ 4 eingestellt. Rührkessel-Kaskaden sind möglich. Der oder die Rührkessel können auf Temperaturen von 20 °C bis 150 °C temperiert werden, wobei die Substanzen in flüssigem Zustand bleiben. Im Phasentrennbehälter (2) wird die wässrige Phase W1 abgetrennt, bevor die organische Phase O1 auf die Membran (3) trifft. Das oxidierte Produkt P kann als Permeat abgetrennt werden. Das Katalysatorsystem C wird in der organischen Phase 02 in einen weiteren Rührkessel (4) geleitet, in den eine wässrige Base B, vorzugsweise Natronlauge, gegeben wird. Die einzelnen Phasen enthalten üblicherweise:
W1: Wasser, Peroxid, Katalysatorsystem, Phasentransferreagenz
O1: organische Verbindung, Produkt, ggf. organisches Lösemittel, Katalysatorsystem, Phasentransferreagenz
O2: wie O1, jedoch höhere Konzentration an Katalysatorsystem und geringere Konzentration an Produkt.

Durch die Abtrennung der wässrigen Phase W1 wird dem Verfahren ein Teil des Katalysatorsystems entnommen. Dieser Anteil des Katalysatorsystems kann durch eine weitere Grundoperation anteilig zurückgewonnen werden und in das ursprüngliche Verfahren zurückgeführt werden. Zum Beispiel eignen sich Membranen in der wässrigen Phase dazu, das Katalysatorsystem anteilig im Retentat zurückzuhalten. Allerdings wird ein Teil des Katalysatorsystems mit dem Permeat dem Verfahren entnommen. Hierdurch verringert sich die Konzentration an Katalysator, so dass der Umsatz der Oxidationsreaktion herabsinken kann. Aus diesem Grund muss frischer Katalysator zugegeben werden, um die Katalysatorkonzentration im Reaktionsgemisch zumindest konstant zu halten.

In einer ersten bevorzugten Variante (Fig. 1) wird die Mischung aus 02 und B wieder dem Rührkessel (1) zugeführt. Bei Bedarf wird dem Rührkessel (1) Säure A zur Einstellung des pH-Werts zugeführt.

In einer zweiten bevorzugten Variante (Fig. 2) wird die Mischung aus 02 und B in einen weiteren Rührkessel (5) geleitet. Dort wird die Mischung mit einer anorganischen Säure A auf den gewünschten pH-Wert eingestellt (bevorzugt 1,5 bis 4) und wieder dem Rührkessel (1) zugeführt.

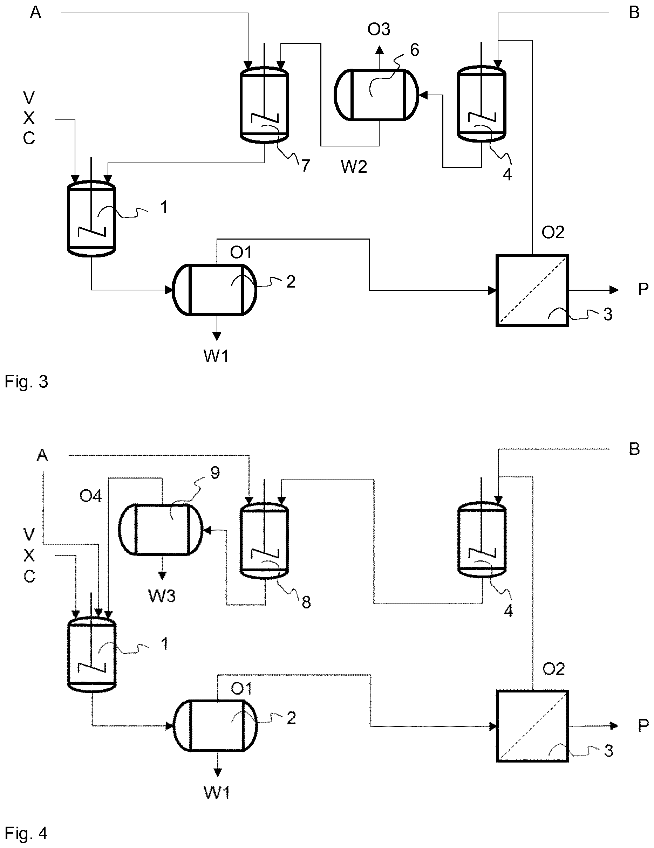

In einer dritten bevorzugten Variante (Fig. 3) wird die Mischung aus 02 und B in einen Phasentrennbehälter (6) gegeben, mit dem die wässrige Phase W2 von der organischen Phase 03 abgetrennt wird. W2 wird in einen Rührkessel (7) geführt, in dem weiterhin die Säure A eingebracht und auf den gewünschten pH-Wert eingestellt (bevorzugt 1,5 bis 4) wird. Die Mischung aus W2 und A wird dem Rührkessel (1) im Kreislauf wieder zugeführt.
W2: Wasser, Base, Katalysatorsystem
O3: wie 02, jedoch ohne Katalysatorsystem

Alternativ kann die dritte Variante ohne Membran (3) durchgeführt werden. Hierbei wird O1 direkt in den Rührkessel (4) überführt. Das Produkt verbleibt in der organischen Phase und kann als 03 abgetrennt werden.

Fig. 4 demonstriert eine vierte bevorzugte Variante, in der die Mischung aus 02 und B in einen Rührkessel (8) überführt und mit der Säure A vermischt wird. Mit der Säure wird auf den gewünschten pH-Wert eingestellt (bevorzugt 1,5 bis 4). Die resultierende Mischung aus 02, B und A gelangt in einen Phasentrennbehälter (9), in dem die wässrige Phase W3 abgetrennt und die verbleibende organische Phase 04 dem Rührkessel (1) im Kreislauf wieder zugeführt wird. Auf Grund der Abtrennung der wässrigen Phase W3 kann weiterhin Säure A in den Rührkessel 1 geleitet werden.
W4: Wasser, Salz der anorganischen Säure und der Base
O4: wie 02.

Die genannten Phasen können weitere Bestandteile enthalten; insbesondere erfolgen Abtrennungen in der Regel nicht quantitativ. Die anorganische Säure A wird vorzugsweise ausgewählt aus Phosphorsäure, Schwefelsäure und Mischungen daraus, wobei Schwefelsäure besonders bevorzugt ist.

Mit dem erfindungsgemäßen Verfahren ist es möglich, homogene Katalysatoren nach Katalyse von Oxidationsreaktionen zurückzugewinnen. Dies ermöglicht eine besonders wirtschaftliche Reaktionsführung, da der Katalysator zurückgewonnen und reaktiviert werden kann. Eine Zugabe an frischem Katalysator ist nur noch im geringen Maß erforderlich.

Ein weiterer Gegenstand der Erfindung ist ein Verfahren zur Epoxidierung von ungesättigten organischen Verbindungen. Hierbei wird ein homogenes Oxidations-Katalysatorsystem eingesetzt, das nach dem zuvor geschriebenen Verfahren reaktiviert worden ist. Das Katalysatorsystem umfasst mindestens ein Derivat eines Metalls in seiner höchsten Oxidationsstufe, wobei das Derivat ausgewählt ist aus H₂WO₄ und H₂MoO₄ bzw. deren Alkali- oder Erdalkalisalzen, und Phosphorsäure. Die Reaktivierung des Katalysatorsystems erfolgt durch Zugabe mindestens einer wässrigen Base auf einen pH-Wert ≥ 4, vorzugsweise ≥ 4,5, bevorzugt ≥ 7, besonders bevorzugt ≥ 11.

Ein weiterer Gegenstand der Erfindung ist ein Verfahren zur Synthese von Lactamen, vorzugsweise Laurinlactam (erfindungsgemäßes Lactamverfahren). In einer ersten Stufe wird die organische Verbindung in Form einer cyclischen, ungesättigten Verbindung, vorzugsweise C6- bis C12-Verbindung, bevorzugt C12-Verbindung, besonders bevorzugt CDEN, zum Epoxid oxidiert. Die Oxidation erfolgt in Gegenwart des homogenen Oxidations-Katalysatorsystems, das nach dem oben genannten Verfahren reaktiviert wird.

Die epoxidierten Verbindungen können im Anschluss in Anwesenheit von einem Katalysator umfassend ein Edelmetall und ein Metalloxid zum entsprechenden Keton umgesetzt werden. Während der Umlagerung oder im Anschluss dessen kann Wasserstoff zugegeben werden, womit das Alkohol-Derivat entstehen kann. Sofern das Keton in einem Gemisch mit dem Alkohol-Derivat vorliegt, kann eine Dehydrierung des Alkohols zum Keton erfolgen. Anschließend kann das Keton oximiert werden. Im Folgeschritt kann die Beckmann-Umlagerung zum Lactam erfolgen, wobei die Umlagerung mittels Schwefelsäure oder Cyanurchlorid durchgeführt kann. Die Lactame können unter Polykondensation zu Polyamiden weiterverarbeitet werden.

Die Umlagerung, die Dehydrierung, die Oximierung, die Beckmann-Umlagerung sowie die Kondensationsreaktion sind dem Fachmann bekannt.

In einer bevorzugten Ausführungsform des erfindungsgemäßen Lactamverfahrens wird aus CDANepoxid Laurinlactam hergestellt, das unter Polymerisation zu Polyamid 12 reagiert.

Im Rahmen des bevorzugten Lactamverfahrens kann CDEN gewonnen werden durch folgende Reaktionsschritte: 1,3-Butadien wird durch Cyclotrimerisation zu Cyclododecatrien umgesetzt. Anschließend erfolgt eine Hydrierung zum Cyclododecen.

### Beispiele

### Beispiel 1 (nicht erfindungsgemäß)

### Kontinuierliche Verfahrensweise ohne Reaktivierung

Es wurde eine Epoxidierung cyclischer, ungesättigter C12-Verbindungen in einer 3-stufigen Rührkesselkaskade kontinuierlich durchgeführt. Die verwendete Rührkessel-Kaskade beinhaltete 2 Reaktoren mit einem Nennvolumen von je 5 Litern und als letzte Stufe einen Rührkessel mit einem Nennvolumen von 25 Litern. Die drei Reaktoren waren mit einem Mantel versehen und wurden über diesen auf eine Temperatur von 80 °C temperiert.

2 kg/h einer cyclischen, ungesättigten C12-Verbindung (91 Gew.-% CDEN und 9 Gew.-% CDAN), Trioctylammoniummethyl-sulfat (als Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 1,05 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. mit einer nominalen Membranfläche von 0,6 m² eingesetzt. Die aktive Trennschicht der Membranen besteht aus Siliconacrylat und die Trägerschicht besteht aus Polyimid.

83% des Feeds zur Membrananlage wurde als Permeat gewonnen. Das Retentat (17% des Feeds zur Membranlage), welches das Katalysatorsystem enthält, wurde zurück in die Reaktion geführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembran Desal DK von GE Power & Water mit einer nominalen Membranfläche von 0,7 m² eingesetzt. 55% des Feeds zur Membrananlage wurde als Permeat gewonnen. Das Retentat (45% des Feeds zur Membranlage), welches das Katalysatorsystem enthält, wurde zurück in den ersten Reaktor der Kaskade geführt.

Zusätzlich zu der Rückführung des Katalysatorsystems mit dem Retentat aus der Membrananlage wurde Phasentransferreagenz, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Trialkylammoniummethyl-su lfat |
|---|---|---|---|
| Menge bezogen auf zugeführte CDEN-Menge | 0,9 mg/g | 3,4 mg/g | 2,2 mg/g |

Mit diesen Katalysator-Kennzahlen ergab sich nach einer Betriebszeit von 105 Stunden ein Umsatz für den CDEN-Anteil von 97% und es wurde eine Konzentration an Wolfram im ersten Reaktor der Kaskade von 0,45 mol-% Wolfram bezogen auf CDEN im Zulauf über ICP-Massenspektrometrie ermittelt.

Nach einer Betriebszeit der Versuchsanlage von 863 Stunden wurde ein Abfall des Umsatzes an CDEN auf 90% festgestellt. Die Konzentration an Wolfram im ersten Reaktor der Kaskade von 0,45 mol-% Wolfram bezogen auf CDEN im Zulauf über ICP-Massenspektrometrie ermittelt. Dementsprechend ergab sich bei gleicher Wolframkonzentration ein signifikant schlechterer Umsatz.

### Beispiel 2 (nicht erfindungsgemäß)

### Kontinuierliche Verfahrensweise ohne Reaktivierung

Es wurde eine Epoxidierung cyclischer, ungesättigter C12-Verbindungen in einer 3-stufigen Rührkesselkaskade kontinuierlich durchgeführt. Die verwendete Rührkessel-Kaskade beinhaltete 2 Reaktoren mit einem Nennvolumen von je 5 Litern und als letzte Stufe einen Rührkessel mit einem Nennvolumen von 25 Litern. Die drei Reaktoren waren mit einem Mantel versehen und wurden über diesen auf eine Temperatur von 80 °C temperiert.

1,5 kg/h einer cyclischen, ungesättigten C12-Verbindung (81 Gew.-% CDEN und 19,1 Gew.-% CDAN), Alamin (Trioctylamin als Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 1,08 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. mit einer nominalen Membranfläche von 0,6 m² eingesetzt. Die aktive Trennschicht der Membranen besteht aus Siliconacrylat und die Trägerschicht besteht aus Polyimid.

79% des Feeds zur Membrananlage wurde als Permeat gewonnen. Das Retentat (21% des Feeds zur Membranlage), welches das Katalysatorsystem enthält, wurde zurück in die Reaktion geführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembran Desal DK von GE Power & Water mit einer nominalen Membranfläche von 0,7 m² eingesetzt. 70% des Feeds zur Membrananlage wurde als Permeat gewonnen. Das Retentat (30% des Feeds zur Membranlage), welches das Katalysatorsystem enthält, wurde zurück in den ersten Reaktor der Kaskade geführt.

Zusätzlich zu der Rückführung des Katalysatorsystems mit den Retentaten aus den Membrananlagen wurden Alamin, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Alamin |
|---|---|---|---|
| Menge bezogen auf zugeführte CDEN-Menge | 2,39 mg/g | 4,4 mg/g | 4,6 mg/g |

Mit diesen Katalysator-Kennzahlen ergab sich ein Gesamtumsatz für den CDEN-Anteil von 94,5%.

Nach einer Betriebszeit der Versuchsanlage von 240 Stunden wurde eine Konzentration an Wolfram im ersten Reaktor der Kaskade von 1,08 mol-% Wolfram bezogen auf CDEN im Zulauf über ICP-Massenspektrometrie ermittelt. Zusätzlich wurde Feststoffablagerungen in der Versuchsanlage beobachtet, die dazu führten, dass die Verbindungsleitungen zwischen Phasentrennbehälter und Membrananlage verstopften und ein weiterer Betrieb der Versuchsanlage nicht mehr möglich war.

### Beispiel 3 (erfindungsgemäß)

### Kontinuierliches Verfahren mit Reaktivierung

Im erfindungsgemäßen Beispiel wurde der Versuchsaufbau aus Beispiel 2 um eine Laugenbehandlung für das Retentat und eine anschließenden Säurezugabe ergänzt (analog Fig. 2).

2,3 kg/h einer cyclischen, ungesättigten C12-Verbindung (91 Gew.-% CDEN und 9 Gew.-% CDAN), Alamin (Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 0,995 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. mit einer nominalen Membranfläche von 0,6 m² eingesetzt. Die aktive Trennschicht der Membranen besteht aus Siliconacrylat und die Trägerschicht besteht aus Polyimid.

79% des Feeds zur Membrananlage wurde als Permeat gewonnen. Das Retentat (21% des Feeds zur Membranlage), welches das Katalysatorsystem enthält, wurde in einen weiteren Rührkessel geführt, in den eine 0,5 M Natronlauge zugegeben wurde. Die Mischung wurde auf einen pH-Wert von 11 eingestellt. Anschließend wurde die Mischung in einen weiteren Rührkessel geleitet und dort mit Schwefelsäure versetzt und auf einen pH von 2 eingestellt. Die resultierende Mischung wurde wieder dem ersten Reaktor der Kaskade zugeführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembran Desal DK von GE Power & Water mit einer nominalen Membranfläche von 0,7 m² eingesetzt. 70% des Feeds zur Membrananlage wurde als Permeat gewonnen. Das Retentat (30% des Feeds zur Membranlage), welches das Katalysatorsystem enthält, wurde zurück in den ersten Reaktor der Kaskade geführt.

Zusätzlich zu der Rückführung des Katalysatorsystems wurden Alamin, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Alamin |
|---|---|---|---|
| Menge bezogen auf zugeführte CDEN-Menge | 1,2 mg/g | 2,0 mg/g | 6 mg/g |

Mit diesen Katalysator-Kennzahlen ergab sich ein Gesamtumsatz für den CDEN-Anteil von 95%.

Nach einer Betriebszeit der Versuchsanlage von 840 Stunden wurde eine Konzentration an Wolfram im ersten Reaktor der Kaskade von 0,51 mol-% Wolfram bezogen auf CDEN im Zulauf über ICP Massenspektrometrie ermittelt. Es wurde keine Feststoffablagerungen in der Versuchsanlage beobachtet, die zu einer Abstellung der Versuchsanlage zwingen würden.

### Beispiel 4 (erfindungsgemäß)

### Kontinuierliches Verfahren mit Reaktivierung

Im erfindungsgemäßen Beispiel wurde der Versuchsaufbau aus Beispiel 3 um eine Phasentrennung nach der Laugenbehandlung für das Retentat ergänzt (analog Fig. 4).

Die organische Phase aus der zusätzlichen Phasentrennung wurde in die Reaktorkaskade zurückgeführt. Die wässrige Phase wurde aus dem Prozess ausgeschleust.

2,3 kg/h einer cyclischen, ungesättigten C12-Verbindung (91 Gew.-% CDEN und 9 Gew.-% CDAN), Alamin (Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 0,979 mol H₂O₂ pro mol CDEN zugegeben.

Das aus zwei flüssigen Phasen bestehende Reaktionsgemisch gelangte von der Kaskade in einen ersten Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. mit einer nominalen Membranfläche von 0,6 m² eingesetzt. Die aktive Trennschicht der Membranen besteht aus Siliconacrylat und die Trägerschicht besteht aus Polyimid.

84% des Feeds zur Membrananlage wurde als Permeat gewonnen. Das Retentat (16% des Feeds zur Membranlage), welches das Katalysatorsystem enthält, wurde in einen weiteren Rührkessel geführt, in den eine 0,5 M Natronlauge zugegeben wurde. Die Mischung wurde auf einen pH-Wert von 11 eingestellt. Anschließend wurde die Mischung in einen weiteren Rührkessel geleitet und dort mit Schwefelsäure versetzt und auf einen pH von 2 eingestellt. Anschließend wurde die Mischung einem zusätzlichen Phasentrennbehälter zugeführt. Die wässrige Phase wurde aus dem Prozess ausgeschleust. Die resultierende organische Phase wurde wieder dem ersten Reaktor der Kaskade zugeführt.

Die wässrige Phase aus dem ersten Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembran Desal DK von GE Power & Water mit einer nominalen Membranfläche von 0,7 m² eingesetzt. 83% des Feeds zur Membrananlage wurde als Permeat gewonnen. Das Retentat (17% des Feeds zur Membranlage), welches das Katalysatorsystem enthält, wurde zurück in den ersten Reaktor der Kaskade geführt.

Zusätzlich zu der Rückführung des Katalysatorsystems wurden Alamin, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Alamin |
|---|---|---|---|
| Menge bezogen auf zugeführte CDEN-Menge | 1,2 mg/g | 3,5 mg/g | 4 mg/g |

Mit diesen Katalysator-Kennzahlen ergab sich ein Gesamtumsatz für den CDEN-Anteil von 91%.

Nach einer Betriebszeit der Versuchsanlage von 500 Stunden wurde eine Konzentration an Wolfram im ersten Reaktor der Kaskade von 0,42 mol-% Wolfram bezogen auf CDEN im Zulauf über ICP Massenspektrometrie ermittelt. Es wurde keine Feststoffablagerungen in der Versuchsanlage beobachtet, die zu einer Abstellung der Versuchsanlage zwingen würden.

### Beispiel 5 (erfindungsgemäß)

### Kontinuierliches Verfahren mit Reaktivierung

Im erfindungsgemäßen Beispiel wurde der Versuchsaufbau aus Beispiel 3 verwendet.

2,0 kg/h einer cyclischen, ungesättigten C12-Verbindung (91 Gew.-% CDEN und 9 Gew.-% CDAN), Trioctylammoniummethyl-sulfat (Phasentransferreagenz), Natriumwolframat und Phosphorsäure und eine 50 %ige H₂O₂-Lösung wurden dem ersten Reaktor der Kaskade zugeführt. Zusätzlich erfolgte eine weitere H₂O₂-Dosierung auf den 2. Reaktor. In Summe wurde ein Verhältnis von 1 mol H₂O₂ pro mol CDEN zugegeben.

Das aus zwei flüssigen Phasen bestehende Reaktionsgemisch gelangte von der Kaskade in einen Phasentrennbehälter, aus dem die organische Phase mittels Pumpe einer kontinuierlichen Membrananlage zugeführt wurde.

Die organischen Phase wurde mit 45°C, einer transmembranen Druckdifferenz von 41,5 bar und einer Überströmung von ca. 300 L/h über die Membranen geführt. Als Membran wurde eine Polymermembran von Evonik MET Ltd. mit einer nominalen Membranfläche von 0,6 m² eingesetzt. Die aktive Trennschicht der Membranen besteht aus Siliconacrylat und die Trägerschicht besteht aus Polyimid.

84% des Feeds zur Membrananlage wurde als Permeat gewonnen. Das Retentat (16% des Feeds zur Membranlage), welches das Katalysatorsystem enthält, wurde in einen weiteren Rührkessel geführt, in den eine 0,5 M Natronlauge zugegeben wurde. Die Mischung wurde auf einen pH-Wert von 11 eingestellt. Anschließend wurde die Mischung in einen weiteren Rührkessel geleitet und dort mit Schwefelsäure versetzt und auf einen pH von 2 eingestellt. Die resultierende Mischung wurde wieder dem ersten Reaktor der Kaskade zugeführt.

Die wässrige Phase aus dem Phasentrennbehälter wurde ebenfalls mittels Pumpe einer zweiten kontinuierlichen Membrananlage zugeführt. Die wässrige Phase wurde mit 43°C, einer transmembranen Druckdifferenz von 40 bar und einer Überströmung von ca. 800 L/h über die Membranen geführt. Als Membran wurde eine Dünnschicht-Komposit Polymermembran Desal DK von GE Power & Water mit einer nominalen Membranfläche von 0,7 m² eingesetzt. 55% des Feeds zur Membrananlage wurde als Permeat gewonnen. Das Retentat (45% des Feeds zur Membranlage), welches das Katalysatorsystem enthält, wurde zurück in den ersten Reaktor der Kaskade geführt.

Zusätzlich zu der Rückführung des Katalysatorsystems wurden Trioctylammoniummethyl-sulfat, Natriumwolframat und Phosphorsäure zum ersten Reaktor gegeben. Die zugegebenen Mengen sind bezogen auf die CDEN-Menge im Feed in der folgenden Tabelle dargestellt.

| | Na₂WO₄ | H₃PO₄ | Trioctylammoniummethyl-sulfat |
|---|---|---|---|
| Menge bezogen auf zugeführte CDEN-Menge | 0,6 mg/g | 3,5 mg/g | 1,6 mg/g |

Mit diesen Katalysator-Kennzahlen ergab sich ein Gesamtumsatz für den CDEN-Anteil von 95%.

Nach einer Betriebszeit der Versuchsanlage von 790 Stunden wurde eine Konzentration an Wolfram im ersten Reaktor der Kaskade von 0,52 mol-% Wolfram bezogen auf CDEN im Zulauf über ICP Massenspektrometrie ermittelt. Es wurde keine Feststoffablagerungen in der Versuchsanlage beobachtet, die zu einer Abstellung der Versuchsanlage zwingen würden.

### Vergleich der Beispiele

Die genannten Beispiele 1-5 und die ergänzende Übersicht zu den Beispielen verdeutlichen die Wirkungsweise der Erfindung.

Beispiel 1 zeigt eine nicht erfindungsgemäße Oxidation von Cyclododecen mit Wasserstoffperoxid, bei der die Wolframkonzentration über der gesamten Versuchszeit konstant gehalten wurde. Der erreichte Umsatz nahm im Versuchsverlauf von 97% nach 105 Stunden auf 90% nach 863 Stunden deutlich ab.

Im Gegensatz dazu wurde im Beispiel 5 der Katalysator durch Zugabe von Natronlauge zum Retentat der organophilen Nanofiltration reaktiviert. Es konnte nach 790 Stunden Betriebszeit im Beispiel 5 bei analogen Betriebsbedingungen und analoger WolframKonzentration ein mit 95% deutlich höherer Umsatz festgestellt werden.

Der Vergleich der Beispiele 1 und 5 zeigt, dass nach rund 800 Betriebsstunden ein höherer Umsatz von CDEN mit weniger Na₂WO₄ und weniger Phasentransferkatalysator erreicht werden kann, wenn der Katalysator reaktiviert wird.Im Beispiel 2 wurde in einer nicht erfindungsgemäßen Ausführung als wesentlicher Unterschied zum Beispiel 1 Alamin anstelle von Trialkylammoniummethylsulfat als Phasentransferreagenz eingesetzt. Ohne Katalysatorreaktivierung musste im Beispiel 2 sehr viel Katalysator frisch zugegeben werden, um gute Umsätze zu erreichen. Es wurde eine sehr hohe Wolfram-Konzentration festgestellt. Zusätzlich erschwerten Feststoffablagerungen den Betrieb der Versuchsanlage und der Versuch musste abgebrochen werden.

In Gegensatz zum Beispiel 2 wurde im Beispiel 3 der Katalysator erfindungsgemäß durch Behandlung des Retentats der organophilen Nanofiltration mit Natronlauge reaktiviert. Es ergaben sich im Beispiel 3 im Vergleich zu Beispiel 2 bei analogem Phasentransferreagenz, größerem Cyclododecen-Durchsatz und längerer Versuchszeit vergleichbare Umsätze und somit ein deutlich höhere Raum-Zeit-Ausbeute. Gleichzeitig wurde im Beispiel 3 weniger frischer Katalysator benötigt und es wurde eine sehr viel niedrigere Wolframkonzentration festgestellt.

Beispiel 4 verdeutlicht eine weitere Ausführungsform der Erfindung - siehe Fig. 4. Im Gegensatz zu Beispiel 3 wird im Beispiel 4 die Wasserphase, die sich nach Reaktivierung der Katalysators im Retentat der organophilen Nanofiltration mit Lauge und anschließender Stellung des pH-Wertes mit Schwefelsäure ergibt, abgetrennt und aus dem Prozess ausgeschleust.

Der Vergleich der Beispiele 2 und 3 zeigt, dass mehr CDEN mit weniger Na₂WO₄ umgesetzt werden kann, wenn der Katalysator reaktiviert wird. Das gleiche gilt für den Vergleich der Beispiele 2 und 4. Außerdem zeigt das Beispiel 2, dass die erhöhte Katalysatormenge, die für die Erreichung des Umsatzes ohne Katalysatorreaktivierung gebraucht wird, zu Verstopfungen und Ausfall der Anlage führen kann.

| **Beispiel** | **Kat- Reak tivie rung** | **PTR** | **Aufbau gemäß Figur** | **Kat- Zugabe in mg/g** | **Versuchsergebnis** | | | **besondere Vorkomm nisse** |
|---|---|---|---|---|---|---|---|---|
| | | | | | **Betriebs stunden** | **gemes sene Wolfram Konzent ration** | **Umsatz** | |
| 1* | nein | TOAMS | - | 0,9 | 105 | 0,45% | 97% | |
| | | | | | 863 | 0,45% | 90% | |
| 2* | nein | Alamin | - | 2,4 | 240 | 1,08 % | 94,5% | Feststoffablagerungen |
| 3 | ja | Alamin | 2 | 1,2 | 840 | 0,51 % | 95% | |
| 4 | ja | Alamin | 4 | 1,2 | 500 | 0,42% | 91% | |
| 5 | ja | TOAMS | 2 | 0,6 | 790 | 0,52% | 95% | |

| | | | | | | | | |
|---|---|---|---|---|---|---|---|---|
| * nicht erfindungsgemäß Kat = Katalysator PTR = Phasentransferreagenz TOAMS = Trioctylammoniummethyl-sulfat | | | | | | | | |

## Patentansprüche

1. Verfahren zur Reaktivierung eines Katalysatorsystems, wobei
• eine ungesättigte organische Verbindung in einem Reaktionsgemisch epoxidiert wird,
• das Reaktionsgemisch ein homogenes Katalysatorsystem zur Epoxidation der ungesättigten organischen Verbindung und mindestens ein Peroxid umfasst,
• das Katalysatorsystem mindestens ein Derivat eines Metalls in seiner höchsten Oxidationsstufe umfasst, wobei das Derivat ausgewählt ist aus H₂WO₄ und H₂MoO₄ bzw. deren Alkali- oder Erdalkalisalze, und Phosphorsäure oder deren Salz, und
• das Katalysatorsystem vor der Reaktivierung von der epoxidierten organischen Verbindung abgetrennt und das Katalysatorsystem nach der Reaktivierung wieder dem Reaktionsgemisch zugeführt wird,
**dadurch gekennzeichnet, dass** die Reaktivierung des Katalysatorsystems durch Zugabe mindestens einer wässrigen Base auf einen pH-Wert ≥ 4 erfolgt.

2. Verfahren nach Anspruch 1, **dadurch gekennzeichnet, dass** das Reaktionsgemisch eine wässrige Phase und eine organische Phase sowie ein Phasentransferreagenz umfasst.

3. Verfahren nach Anspruch 2, **dadurch gekennzeichnet, dass** die Oxidation bei einem pH-Wert ≤ 4 erfolgt.

4. Verfahren nach Anspruch 3, **dadurch gekennzeichnet, dass** zur Einstellung des pH-Werts ≤ 4 mindestens eine anorganische Säure mit einem pKₛ-Wert (25 °C) von 2,5 oder kleiner ausgewählt wird.

5. Verfahren nach einem der vorherigen Ansprüche, **dadurch gekennzeichnet, dass** der pH-Wert des Katalysatorsystems nach der Reaktivierung und vor der Zuführung auf einen Wert von < 7 eingestellt wird.

6. Verfahren nach einem der vorherigen Ansprüche, **dadurch gekennzeichnet, dass** eine ggf. vorhandene organische Phase nach der Reaktivierung und vor der Zuführung zum Reaktionsgemisch abgetrennt wird.

7. Verfahren nach einem der vorherigen Ansprüche, **dadurch gekennzeichnet, dass** als Peroxid Wasserstoffperoxid eingesetzt wird.

8. Verfahren nach einem der vorherigen Ansprüche, **dadurch gekennzeichnet, dass** die Base ausgewählt ist aus Ammoniak, Alkalihydroxiden oder Mischungen daraus.

9. Verfahren nach Anspruch 8, **dadurch gekennzeichnet, dass** das Alkalimetall aus Natrium und Kalium ausgewählt ist.

10. Verfahren nach einem der vorherigen Ansprüche, **dadurch gekennzeichnet, dass** die organische Verbindung eine ungesättigte cyclische Verbindung mit sechs bis zwölf Kohlenstoffatomen ist.

11. Verfahren nach einem der vorherigen Ansprüche, **dadurch gekennzeichnet, dass** die Epoxidation ohne organisches Lösemittel erfolgt.

12. Verfahren zur Epoxidation von ungesättigten organischen Verbindungen mittels Peroxiden, wobei ein homogenes Epoxidations-Katalysatorsystem eingesetzt wird, welches mindestens ein Derivat eines Metalls in seiner höchsten Oxidationsstufe umfasst, wobei das Derivat ausgewählt ist aus H₂WO₄ und H₂MoO₄ bzw. deren Alkali- oder Erdalkalisalzen, und Phosphorsäure oder deren Salz, **dadurch gekennzeichnet, dass** eine Reaktivierung des Katalysatorsystems durch Zugabe mindestens einer wässrigen Base auf einen pH-Wert ≥ 4, erfolgt und das Katalysatorsystem vor der Reaktivierung von der epoxidierten organischen Verbindung abgetrennt und das Katalysatorsystem nach der Reaktivierung wieder dem Reaktionsgemisch zugeführt wird.

13. Verfahren nach Anspruch 12, **dadurch gekennzeichnet, dass** die Reaktivierung des Katalysatorsystems durch Zugabe mindestens einer wässrigen Base auf einen pH-Wert ≥ 4,5 erfolgt.

14. Verfahren nach Anspruch 12, **dadurch gekennzeichnet, dass** die Reaktivierung des Katalysatorsystems durch Zugabe mindestens einer wässrigen Base auf einen pH-Wert ≥ 7 erfolgt.

15. Verfahren zur Synthese von Lactamen, umfassend die Schritte
• Epoxidation einer cyclischen, ungesättigten Verbindung zum Epoxid,
• Umlagerung zu Keton,
• Oximierung zum Oxim und
• Umlagerung zum Lactam,
wobei die Epoxidation der cyclischen, ungesättigten Verbindung in Gegenwart eines homogenen Oxidations-Katalysatorsystems erfolgt, **dadurch gekennzeichnet, dass** eine Reaktivierung des Katalysatorsystems nach einem Verfahren der Ansprüche 1 bis 11 erfolgt.

## Claims

1. Method for reactivating a catalyst system wherein
• an unsaturated organic compound is epoxidized in a reaction mixture,
• the reaction mixture comprises a homogeneous catalyst system for the epoxidation of the unsaturated organic compound and at least one peroxide,
• the catalyst system comprises at least one derivative of a metal in its highest oxidation state, wherein the derivative is selected from H₂WO₄ and H₂M₀O₄ or the alkali metal and alkaline earth metal salts thereof, and phosphoric acid or the salt thereof, and
• the catalyst system is separated from the epoxidized organic compound prior to the reactivation and the catalyst system after the reactivation is recycled to the reaction mixture,
**characterized in that** the catalyst system is reactivated by addition of at least one aqueous base at a pH ≥ 4.

2. Method according to Claim 1, **characterized in that** the reaction mixture comprises an aqueous phase and an organic phase and also a phase transfer reagent.

3. Method according to Claim 2, **characterized in that** the oxidation is carried out at a pH ≤ 4.

4. Method according to Claim 3, **characterized in that** to adjust the pH to ≤ 4, at least one inorganic acid having a pKₐ (25°C) of 2.5 or less is selected.

5. Method according to any of the preceding claims, **characterized in that** the pH of the catalyst system after the reactivation and before the feed is adjusted to a value of <7.

6. Method according to any of the preceding claims, **characterized in that** an organic phase optionally present is removed after the reactivation and before the feeding to the reaction mixture.

7. Method according to any of the preceding claims, **characterized in that** the peroxide used is hydrogen peroxide.

8. Method according to any of the preceding claims, **characterized in that** the base is selected from ammonia, alkali metal hydroxides or mixtures thereof.

9. Method according to Claim 8, **characterized in that** the alkali metal is selected from sodium and potassium.

10. Method according to any of the preceding claims, **characterized in that** the organic compound is an unsaturated cyclic compound having six to twelve carbon atoms.

11. Method according to at any of the preceding claims, **characterized in that** the epoxidation takes place without organic solvent.

12. Method for the epoxidation of unsaturated organic compounds by means of peroxides, wherein a homogeneous epoxidation catalyst system is used, which comprises at least one derivative of a metal in its highest oxidation state, wherein the derivative is selected from H₂WO₄ and H₂M₀O₄ or the alkali metal and alkaline earth metal salts thereof, and phosphoric acid or the salt thereof, **characterized in that** the catalyst system is reactivated by addition of at least one aqueous base at a pH ≥ 4 and the catalyst system is separated from the epoxidized organic compound prior to the reactivation and the catalyst system after the reactivation is recycled to the reaction mixture.

13. Method according to Claim 12, **characterized in that** the catalyst system is reactivated by addition of at least one aqueous base at a pH ≥ 4.5.

14. Method according to Claim 12, **characterized in that** the catalyst system is reactivated by addition of at least one aqueous base at a pH ≥ 7.

15. Method for the synthesis of lactams comprising the steps of:
• epoxidation of a cyclic unsaturated compound to the epoxide,
• rearrangement to the ketone,
• oximation to the oxime and
• rearrangement to the lactam,
wherein the cyclic unsaturated compound is epoxidized in the presence of a homogeneous oxidation catalyst system, **characterized in that** a reactivation of the catalyst system is carried out according to a method of Claims 1 to 11.

## Revendications

1. Procédé de réactivation d'un système catalytique, selon lequel
- un composé organique insaturé est époxydé dans un mélange réactionnel,
- le mélange réactionnel comprend un système catalytique homogène pour l'époxydation du composé organique insaturé et d'au moins un peroxyde,
- le système catalytique comprend au moins un dérivé d'un métal à son niveau d'oxydation le plus élevé, le dérivé étant choisi parmi H₂WO₄ et H₂MoO₄ ou leurs sels alcalins ou alcalino-terreux, et l'acide phosphorique ou son sel, et
- le système catalytique est séparé du composé organique époxydé avant la réactivation, et le système catalytique est réintroduit dans le mélange réactionnel après la réactivation,
**caractérisé en ce que** la réactivation du système catalytique a lieu par ajout d'au moins une base aqueuse à un pH ≥ 4.

2. Procédé selon la revendication 1, **caractérisé en ce que** le mélange réactionnel comprend une phase aqueuse et une phase organique, ainsi qu'un réactif de transfert de phases.

3. Procédé selon la revendication 2, **caractérisé en ce que** l'oxydation a lieu à un pH ≤ 4.

4. Procédé selon la revendication 3, **caractérisé en ce qu'**au moins un acide inorganique ayant un pKₛ (25 °C) de 2,5 ou moins est choisi pour l'ajustement du pH ≤ 4.

5. Procédé selon l'une quelconque des revendications précédentes, **caractérisé en ce que** le pH du système catalytique est ajusté à une valeur < 7 après la réactivation et avant l'introduction.

6. Procédé selon l'une quelconque des revendications précédentes, **caractérisé en ce qu'**une phase organique éventuellement présente est séparée après la réactivation et avant l'introduction dans le mélange réactionnel.

7. Procédé selon l'une quelconque des revendications précédentes, **caractérisé en ce que** du peroxyde d'hydrogène est utilisé en tant que peroxyde.

8. Procédé selon l'une quelconque des revendications précédentes, **caractérisé en ce que** la base est choisie parmi l'ammoniac, les hydroxydes alcalins ou leurs mélanges.

9. Procédé selon la revendication 8, **caractérisé en ce que** le métal alcalin est choisi parmi le sodium et le potassium.

10. Procédé selon l'une quelconque des revendications précédentes, **caractérisé en ce que** le composé organique est un composé cyclique insaturé contenant six à douze atomes de carbone.

11. Procédé selon l'une quelconque des revendications précédentes, **caractérisé en ce que** l'époxydation a lieu sans solvant organique.

12. Procédé d'époxydation de composés organiques insaturés au moyen de peroxydes, selon lequel un système catalytique d'époxydation homogène est utilisé, qui comprend au moins un dérivé d'un métal à son niveau d'oxydation le plus élevé, le dérivé étant choisi parmi H₂WO₄ et H₂MoO₄ ou leurs sels alcalins ou alcalino-terreux, et l'acide phosphorique ou son sel, **caractérisé en ce qu'**une réactivation du système catalytique a lieu par ajout d'au moins une base aqueuse à un pH ≥ 4, et le système catalytique est séparé du composé organique époxydé avant la réactivation, et le système catalytique est réintroduit dans le mélange réactionnel après la réactivation.

13. Procédé selon la revendication 12, **caractérisé en ce que** la réactivation du système catalytique a lieu par ajout d'au moins une base aqueuse à un pH ≥ 4,5.

14. Procédé selon la revendication 12, **caractérisé en ce que** la réactivation du système catalytique a lieu par ajout d'au moins une base aqueuse à un pH ≥ 7.

15. Procédé de synthèse de lactames, comprenant les étapes suivantes :
- l'époxydation d'un composé cyclique insaturé pour former l'époxyde,
- le réarrangement pour former la cétone,
- l'oximation pour former l'oxime, et
- le réarrangement pour former le lactame, l'époxydation du composé cyclique insaturé ayant lieu en présence d'un système catalytique d'oxydation homogène,
**caractérisé en ce qu'**une réactivation du système catalytique a lieu par un procédé selon les revendications 1 à 11.
